# DEMANDE DE BREVET EUROPEEN

(11) **EP 1 129 691 A2**
(43) Date de publication de la demande: **05.09.2001**
(21) Numéro de dépôt: 01400448.5
(22) Date de dépôt: 20.02.2001
(51) Int. Cl.: A61K 7/13

(54) **Compositions de teinture des fibres kératiniques contenant des dérives d'indolizine et procédé de teinture**

(30) Priorité: 25.02.2000 FR 0002420
(71) Demandeur: L'OREAL, 75008 Paris (FR)
(72) Inventeur: Breton, Philippe, 78150 Le Chesnay (FR); Lagrange, Alain, 77700 Coupvray (FR)
(74) Mandataire: Fevrier, Murielle

(57) **Abrégé**

L'invention a pour objet une composition pour la teinture d'oxydation des fibres kératiniques contenant au moins un dérivé d'indolizine à titre de coupleur et au moins une base d'oxydation.

L'invention a également pour objet l'utilisation de dérivés d'indolizine à titre de coupleur pour la teinture d'oxydation des fibres kératiniques en association avec au moins une base d'oxydation, ainsi que les procédés de teinture les mettant en oeuvre.

## Description

L'invention a pour objet une composition pour la teinture d'oxydation des fibres kératiniques contenant au moins un dérivé d'indolizine à titre de coupleur et au moins une base d'oxydation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet différemment sensibilisée (i.e. abîmée) entre sa pointe et sa racine.

L'utilisation de composés de type indazole est connue dans le domaine de la coloration des cheveux. La demande de brevet DE-A-1 492 166 décrit la polycondensation par oxydation de tels composés. La demande de brevet DE-A-2 623 564 décrit l'association d'hydroxy indazoles avec des tétraamino pyrimidines. Le brevet US 4 013 404 décrit certains amino indazoles ainsi que leur utilisation à titre de précurseurs de colorants d'oxydation.

La demanderesse vient maintenant de découvrir qu'il est possible d'obtenir de nouvelles teintures puissantes, peu sélectives et particulièrement résistantes, capables d'engendrer des colorations intenses dans des nuances variées, en utilisant des dérivés d'indolizine particuliers.

Cette découverte est à la base de la présente invention.

L'invention a donc pour objet une composition pour la teinture des fibres kératiniques et en particulier des fibres kératiniques humaines telles que les cheveux, caractérisée par le fait qu'elle comprend, dans un milieu approprié pour la teinture :
- à titre de coupleur, au moins un dérivé d'indolizine de formule (I), et/ou au moins l'un de ses sels d'addition avec un acide : 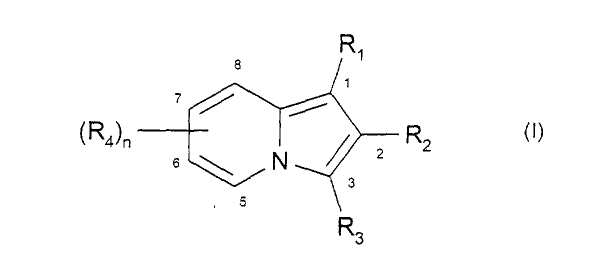 dans laquelle :

- n est un nombre entier compris entre 0 et 4 inclusivement ;
- R₁ et R₃, identiques ou différents, représentent un atome d'hydrogène ou d'halogène ; un radical alkyle en C₁-C₄ ; alcoxy en C₁-C₄ ; monohydroxyalkyle en C₁-C₄ ; polyhydroxyalkyle en C₂-C₄ ; aminoalkyle en C₁-C₄ ; acétylamino ; acétylaminoalkyle en C₁-C₄ ; alcoxy(C₁-C₄)alkyle en C₁-C₄ ; monoalkyl(C₁-C₄)aminoalkyle en C₁-C₄ ; dialkyl(C₁-C₄)aminoalkyle en C₁-C₄ ; aminocarboxyalkyle en C₁-C₄ ; acétyl alkyl(C₁-C₄)aminoalkyle en C₁-C₄ ; ou un cycle aromatique non substitué ou substitué par un ou plusieurs radicaux choisis parmi un atome d'halogène, un radical alkyle en C₁-C₄, trifluorométhyle, alcoxy en C₁-C₄, amino, et amino mono- ou disubstitué par un radical alkyle en C₁-C₄, par un radical alkyl(C₁-C₄)aminoalkyle en C₁-C₄, par un radical dialkyl(C₁-C₄)aminoalkyle en C₁-C₄, par un radical carboxyle ou par un radical sulfoxy ;
   R₃ peut également représenter un hétérocyclique insaturé à 5 ou 6 chaînons ;
- R₂ et R₄, identiques ou différents, représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄, monoalkyl(C₁-C₄)aminoalkyle en C₁-C₄, dialkyl(C₁-C₄)aminoalkyle en C₁-C₄, carboxy, cyano, carboxyalkyle en C₁-C₄, nitro, ou un cycle aromatique à 5 ou 6 chaînons non substitué ou substitué par un ou plusieurs radicaux choisis parmi un atome d'halogène, un radical alkyle en C₁-C₄, trifluorométhyle, alcoxy en C₁-C₄, amino, et amino mono- ou disubstitué par un radical alkyle en C₁-C₄, par un radical alkyl(C₁-C₄)aminoalkyle en C₁-C₄, par un radical dialkyl(C₁-C₄)aminoalkyle en C₁-C₄, par un radical carboxyle ou par un radical sulfoxy ;
étant entendu qu'au moins un des radicaux R₁ et R₃ représente un atome d'hydrogène ;
- et au moins une base d'oxydation.

Dans la formule (I) ci-dessus, les groupements alkyle et alcoxy peuvent être linéaires ou ramifiés.

Parmi les cycles aromatiques de la formule (I) ci-dessus, on peut notamment citer les cycles phényle, nitrophényle, alkylphényle, alcoxyphényle, polyalkylphényle, et polyalcoxyphényle.

Parmi les hétérocycles insaturés à 5 ou 6 chaînons pouvant être représentés par le radical R₃, on peut notamment citer les cycles pyrrolique, pyridinique, pyrimidinique, imidazolique, pyrazolique, oxazolique, thiazolique, triazolique, pyrazolotriazolique, pyrazoloimidazolique, pyrrolotriazolique, pyrazolopyrimidinique, pyrazolopyridinique, benzoimidazolique, benzoxazolique, benzothiazolique, indolique, indolinique, indolidinique, isoindolidinique, benzotriazolinique, pyrazinique, oxazinique, triazinique, quinolinique, tetrahydroquinolinique, benzoimidazolidinique, et benzopyrimidinique.

Les composés de formule (I) sont, pour leur grande majorité, des composés connus, notamment dans le domaine de la photographie, et dont le ou les modes de préparation sont décrits dans les ouvrages suivants :
- Chichibabin, *Ber.* **1927**, 60, 1607 ;
- Borrows et al., J. Chem. Soc., **1946**, 1069 ;
- T. Uchida et al., *Synthesis*, **1976**, 209-236.

Les colorations obtenues avec la composition de teinture conforme à l'invention, sont de nuances variées, puissantes, peu sélectives et présentent d'excellentes propriétés de résistance à la fois vis-à-vis des agents atmosphériques tels que la lumière et les intempéries et vis-à-vis de la transpiration et des différents traitements que peuvent subir les cheveux (shampooings, déformations permanentes, lumière, transpiration, etc...).

Parmi les dérivés d'indolizine de formule (I), utilisables à titre de coupleur dans la composition tinctoriale conforme à l'invention, on peut notamment citer :
- l'indolizine ;
- la 2-méthyl-1-phényl-indolizine ;
- la 2,8-diméthyl-indolizine ;
- l'ester méthylique de l'acide 2-méthyl-indolizine-6-carboxylique ;
- la 2,7-diméthyl-indolizine ;
- la 2-méthyl-1-(4-nitro-phényl)-indolizine ;
- la 8-méthyl-2-phényl-indolizine ;
- la 2-méthyl-indolizine ;
- la 7-méthyl-2-phényl-indolizine ;
- la 2-phényl-indolizine ;
- la 7-méthyl-2-(3-nitro-phényl)-indolizine ;
- la 7-méthyl-2-(2,5-diméthoxyphényl)-indolizine ;
- la 2-(2-pyridinyl)-indolizine ;
- la 2-(4-N,N-diméthylamino-phényl)-indolizine ;
- la 2-phényl-3-[2-(4-pyridyl)éthyl]-indolizine ;
- la 2-phényl-3-[2-(2-pyridyl)éthyl]-indolizine ;
- la 2-éthyl-7-méthoxy-indolizine ;
- la 2-éthyl-8-méthoxy-indolizine ;
- l'éthyl ester d'acide 2-butyl-7-indolizine-carboxylique ;
- la 1-éthyl-indolizine ;
- la 6,8-diméthyl-indolizine ;
- la 5-éthyl-7-méthyl-indolizine ;
- la N,N-diéthyl-2-méthyl-1-indolizine-éthanamine ;
- la N,N-diméthyl-2-méthyl-1-indolizine-éthanamine ;
- la N-éthyl-2-méthyl-1-indolizine-éthanamine ;
- la N-méthyl-2-méthyl-1 -indolizine-éthanamine ;
- la -2-méthyl-1-indolizine-éthanamine ;
- la 5,7-diméthyl-indolizine ;
- la 8-cyano-2-méthyl-indolizine ;
- la 1-éthyl-2-méthyl-indolizine ;
- l'acide 5-indolizineacétique ;
- la dihydro-indolizine ;
- la 1-indolizine-alanine
- la 6-éthyl-1,2-diméthyl-indolizine ;
- la 3-éthyl-indolizine ;
- la 2-éthyl-1-méthyl-indolizine ;
- la N,N-diéthyl-2-méthyl-3-indolizine-éthanamine ;
- la N-éthyl-2-méthyl-3-indolizine-éthanamine ;
- la N-éthyl-N-[2-(2-méthyl-3-indolizinyl)éthyl]-acétamide ;
- la N-[2-(2-méthyl-3-indolizinyl)éthyl]-acétamide ;
- la N,N,2-triméthyl-3-indolizine-éthanamine ;
- la N,2-diméthyl-3-indolizine-éthanamine ;
- la 2-méthyl-3-indolizine-éthanamine ;
- l'éthyl ester d'acide 7-indolizine carboxylique ;
- l'éthyl ester d'acide 6-indolizine carboxylique ;
- la N,N-diéthyl-2-méthyl-3-indolizine-propanamine ;
- la N-éthyl-N-[3-(2-méthyl-3-indolizinyl)propyl]-acétamide ;
- la N-éthyl-2-méthyl-3-indolizine-propanamine ;
- la N-[3-(2-méthyl-3-indolizinyl)propyl]-acétamide ;
- la N,N,2-triméthyl-3-indolizine-propanamine ;
- la N,2-diméthyl-3-indolizine-propanamine ;
- la 2-méthyl-3-indolizine-propanamine ;
- la 2-méthyl-8-nitro-indolizine ;
- la 2-méthyl-6-nitro-indolizine ;
- la N,N'-diméthyl-1,2-indolizine-éthanamine ;
- le méthyl ester d'acide 2-méthyl-7-indolizine-carboxylique ;
- l'éthyl ester d'acide 2-méthyl-7-indolizine-carboxylique ;
- la 2-méthyl-7-indolizine-carboxamide ;
- la N-éthyl-2-indolizine-éthanamine ;
- la N-méthyl-2-indolizine-éthanamine ;
- l'éthyl ester d'acide 2-méthyl-8-indolizine-carboxylique ;
- l'acide 2-méthyl 8-indolizine-carboxylique ;
- la 2-méthyl-6-méthoxy-indolizine ;
- la 2,8-diméthyl-indolizine ;
- la 8-méthyl-indolizine ;
- la N-[(2-méthyl-7-indolizinyl)méthyl]-acétamide ;
- la 7-(aminométhyl)-2-méthyl-indolizine ;
- la 2-méthyl-7-indolizine-carboxamide ;
- la 1-β-hydroxyéthyl-2-méthyl-indolizine ;
- la 6-hydroxyméthyl-2-méthyl-indolizine ;
- l'éthyl ester de 6-carboxy-2-méthyl-indolizine ;
- la 1-hydroxyméthyl-indolizine ;
- la 2-aminométhyl-indolizine ;
- la 2-hydroxyméthyl-indolizine ;
- la 2,3,7-triméthyl-indolizine ;
- la 2,3,6-triméthyl-indolizine ;
- la 5-méthyl-indolizine ;
- la 3,5-diméthyl-indolizine ;
- la 1-méthyl-indolizine ;
- la 2-méthyl-indolizine ;
- la 7-méthyl-indolizine ;
- la 6-méthyl-indolizine ;
- la 3-méthyl-indolizine ;
- la 6-éthyl-2,3-diméthyl-indolizine ;
- la 6-éthyl-2-méthyl-indolizine ;
- la 1,2-diméthyl-indolizine ;
- la 2,7-diméthyl-indolizine
- la 2,6-diméthyl-indolizine
- la 2,5-diméthyl-indolizine ;
- la 2,3-diméthyl-indolizine ;
- la 2-(3-furanyl)-indolizine ;
- la 2-(2-thiazolyl)-indolizine ;
- la 2-(3-thiényl)-indolizine ;
- la 2-(2-thiényl)-indolizine ;
- la 5,7-diméthyl-2-phényl-indolizine ;
- la 2-(3,4-diméthoxyphényl)-indolizine ;
- la 5-carboxy-2-phényl-indolizine ;
- l'acide 4-(7-indolizinyl)-benzène-sulfonique ;
- l'acide 4-(2-indolizinyl)-benzène-sulfonique ;
- la 3,8-diméthyl-2-phényl-indolizine ;
- la 3,7-diméthyl-2-phényl-indolizine ;
- la 3,6-diméthyl-2-phényl-indolizine ;
- la 3,5-diméthyl-2-phényl-indolizine ;
- la 7-méthyl-8-nitro-2-phényl-indolizine ;
- la 7-méthyl-6-nitro-2-phényl-indolizine ;
- la 1-β-aminoéthyl-2-phényl-indolizine ;
- la 8-méthyl-2-phényl-indolizine ;
- la 2-(3-méthoxyphényl)-indolizine ;
- la 6-éthyl-2-phényl-indolizine ;
- la 2-(2,5-diméthoxyphényl)-5-méthyl-indolizine ;
- la 2-(2,5-diméthoxyphényl)-indolizine ;
- la 6-méthyl-2-phényl-indolizine ;
- la 8-carboxy-2-phényl-indolizine ;
- la 2-(4-γ-hydroxypropylphényl)-indolizine ;
- la 2-(4-β-hydroxyéthylphényl)-indolizine ;
- la 8-nitro-2-phényl-indolizine ;
- la 6-nitro-2-phényl-indolizine ;
- la 6-carboxy-2-phényl-indolizine ;
- la 6-carboxy-2-phényl-indolizine ;
- la 5-méthyl-2-phényl-indolizine ;
- la 7-méthoxy-2-phényl-indolizine ;
- la 1-[2-(diméthylamino)éthyl]-2-phényl-indolizine ;
- la 1-[3-(diméthylamino)propyl]-2-phényl-indolizine ;
- la 1-[(diméthylamino)méthyl]-2-phényl-indolizine ;
- la 2-(4-méthoxyphényl)-indolizine ;
et leurs sels d'addition avec un acide.

Le ou les dérivés d'indolizine de formule (I) et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

La nature de la ou des bases d'oxydation utilisées dans la composition tinctoriale conforme à l'invention n'est pas critique. Elles peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans la composition tinctoriale conforme à l'invention, on peut notamment citer les composés de formule (II) suivante et leurs sels d'addition avec un acide : 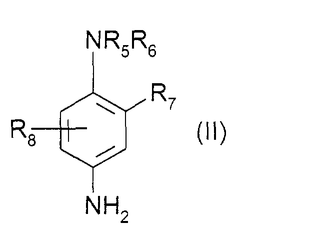 dans laquelle :
- R₅ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₆ représente un atome d'hydrogène, un radical alkyle en C₁-C_{4,} monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₇ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₈ représente un atome d'hydrogène, d'halogène ou un radical alkyle en C₁-C₄.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (II) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (Il) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (III) suivante, et leurs sels d'addition avec un acide : 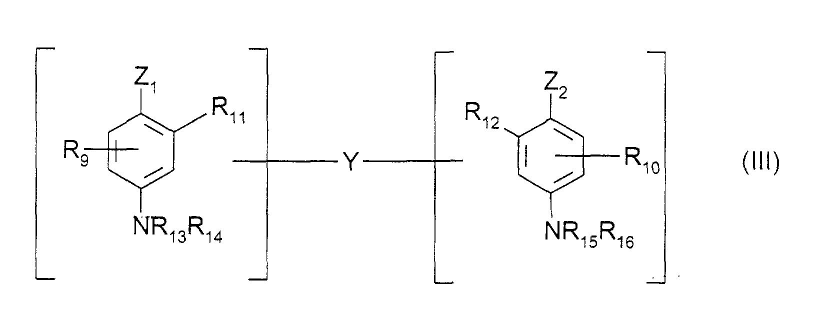 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₉ et R₁₀ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₁, R₁₂, R₁₃, R₁₄, R₁₅ et R₁₆, identiques ou différents, représentent un atome d'hydrogène, un bras de liaison Y ou un radical alkyle en C₁-C₄ ;
étant entendu que les composés de formule (III) ne comportent qu'un seul bras de liaison Y par molécule.

Parmi les groupements azotés de la formule (III) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (III) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (III), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (IV) suivante, et leurs sels d'addition avec un acide : 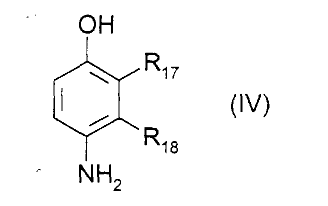 dans laquelle :
- R₁₄ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₈ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₇ ou R₁₈ représente un atome d'hydrogène.

Parmi les para-aminophénols de formule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1-(β-hydroxyéthyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation représentent de préférence de 0,0005 à 12 % en poids environ du poids total de la composition tinctoriale conforme à l'invention, et encore plus préférentiellement de 0,005 à 6 % en poids environ de ce poids.

La composition tinctoriale conforme à l'invention peut également renfermer un ou plusieurs coupleurs additionnels, (différents des dérivés d'indolizine de formule (I)), et/ou un ou plusieurs colorants directs de façon à faire varier ou enrichir en reflets les nuances obtenues.

Parmi les coupleurs additionnels utilisables dans la composition tinctoriale conforme à l'invention, on peut notamment citer les métaphénylènediamines, les méta-aminophénols, les métadiphénols et les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

Ces coupleurs additionnels sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

Les sels d'addition avec un acide, (dérivés d'indolizine de formule (I), bases d'oxydation et coupleurs additionnels), utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol, les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désiréeau moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 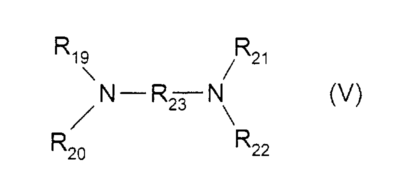 dans laquelle R₂₃ est un reste propylène éventuellement substitué par un groupement hydroxyle ou un radical alkyle en C₁-C₄ ; R₁₉, R₂₀, R₂₁ et R₂₂, identiques ou différents, représentent un atome d'hydrogène, un radical alkyle en C₁-C₄ ou hydroxyalkyle en C₁-C₄.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale conforme à l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des dérivés d'indolizine de formule (I) ci-dessus, à titre de coupleur, en association avec au moins une base d'oxydation, pour la teinture d'oxydation des fibres kératiniques et en particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale telle que définie ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides et les enzymes telles que les peroxydases, les laccases, les tyrosynases et les oxydo-réductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture à plusieurs compartiments ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Enfin certains dérivés d'indolizine de formule (I) sont nouveaux en soi et constituent à ce titre un autre objet de l'invention.

Ces nouveaux dérivés d'indolizine sont les suivants :
- l'ester méthylique de l'acide 2-méthyl-indolizine-6-carboxylique ;
- la 7-méthyl-2-(2,5-diméthoxyphényl)-indolizine ;
et leurs sels d'addition avec un acide.

Les sels d'addition avec un acide des composés nouveaux ci-dessus sont choisis parmi ceux définis précédemment pour les composés de formule (I).

Ces composés particuliers peuvent être préparés selon les procédés de synthèse précédemment indiqué pour les composés de formule (I).

Les exemples qui suivent sont destinés à illustrer l'invention.

### EXEMPLES DE TEINTURE

### EXEMPLES 1 à 5 DE TEINTURE EN MILIEU NEUTRE

On a préparé les compositions tinctoriales conformes à l'invention suivantes :

| **EXEMPLE** | **1** | **2** | **3** | **4** | **5** |
|---|---|---|---|---|---|
| Ester méthylique de l'acide 2-méthylindolizine-6-carboxylique (coupleur de formule (I)) | 3.10⁻³ mole | 3.10⁻³ mole | 3.10⁻³ mole | 3.10⁻³ mole | - |
| 2-méthyl indolizine (coupleur de formule (I)) | - | - | - | - | 3.10⁻³ mole |
| Paraphénylènediamine (base d'oxydation) | 3.10⁻³ mole | - | - | - | - |
| Paratoluylènediamine (base d'oxydation) | - | 3.10⁻³ mole | - | - | - |
| Dichlorhydrate de 3,7-diaminopyrazolopyrimidine (base d'oxydation) | - | - | 3.10⁻³ mole | - | 3.10⁻³ mole |
| Dichlorhydrate de N-(β-méthoxyéthyl) paraphénylènediamine (base d'oxydation) | - | - | - | 3.10⁻³ mole | - |
| Support de teinture commun n°1 | (*) | (*) | (*) | (*) | (*) |
| Eau déminéralisée q.s.p. | 100 g | 100 g | 100 g | 100 g | 100 g |

| | | | | | |
|---|---|---|---|---|---|
| (*) : Support de teinture commun n°1 : - Ethanol 18 g - Tampon phosphate (K₂HPO₄ 1.5M / KH₂PO₄ 1M) 10 g - Métabisulfite de sodium 0,205 g - Séquestrant q.s. | | | | | |

Au moment de l'emploi, on a mélangé poids pour poids chacune des compositions tinctoriales ci-dessus avec une solution de peroxyde d'hydrogène à 20 volumes (6% en poids) et de pH 3.

Chacun des mélanges obtenus présentait un pH d'environ 6,8 et a été appliqué pendant 30 minutes sur des mèches de cheveux gris naturels à 90 % de blancs. Les mèches ont ensuite été rincées, lavées au shampooing, rincées à nouveau puis séchées.

### Les nuances obtenues figurent dans le tableau ci-après :

| **Exemple** | **Nuance obtenue** |
|---|---|
| **1** | Gris très foncé |
| **2** | Vert foncé |
| **3** | Violet |
| **4** | Bleu vert foncé |
| **5** | Blond foncé violacé cendré |

### EXEMPLE 6 DE TEINTURE EN MILIEU ALCALIN

On a préparé la composition tinctoriale conforme à l'invention suivante :

| **EXEMPLE** | **6** |
|---|---|
| 2-méthyl indolizine (coupleur de formule (I)) | 3.10⁻³ mole |
| Dichlorhydrate de 3,7-diaminopyrazolopyrimidine (base d'oxydation) | 3.10⁻³ mole |
| Support de teinture commun n°2 | (**) |
| Eau déminéralisée q.s.p. | 100 g |

| | |
|---|---|
| (**) : Support de teinture commun n°2 : - Ethanol 18 g - Ammoniaque à 20% de NH₃ 10 g - Métabisulfite de sodium 0,205 g - Séquestrant q.s. | |

Au moment de l'emploi, on a mélangé poids pour poids la compositions tinctoriale ci-dessus avec une solution de peroxyde d'hydrogène à 20 volumes (6% en poids) et de pH 3.

Le mélange obtenu présentait un pH d'environ 10 et a été appliqué pendant 30 minutes sur des mèches de cheveux gris naturels à 90 % de blancs. Les mèches ont ensuite été rincées, lavées au shampooing, rincées à nouveau puis séchées.

Les cheveux ont été teints dans une nuance blond cendré irisé.

## Revendications

1. Composition pour la teinture de fibres kératiniques et en particulier des fibres kératiniques humaines telles que les cheveux, caractérisée par le fait qu'elle comprend, dans un milieu approprié pour la teinture :
- à titre de coupleur, au moins un dérivé d'indolizine de formule (I), et/ou au moins l'un de ses sels d'addition avec un acide : 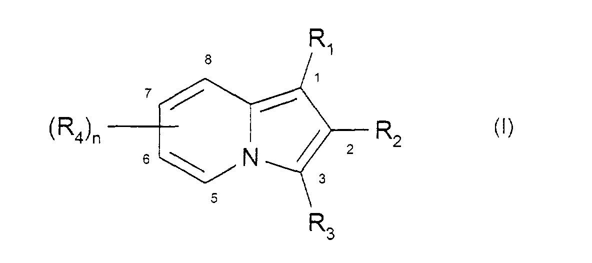 dans laquelle :
- n est un nombre entier compris entre 0 et 4 inclusivement ;
- R₁ et R₃, identiques ou différents, représentent un atome d'hydrogène ou d'halogène ; un radical alkyle en C₁-C₄ ; alcoxy en C₁-C₄ ; monohydroxyalkyle en C₁-C₄ ; polyhydroxyalkyle en C₂-C₄ ; aminoalkyle en C₁-C₄ ; acétylamino ; acétylaminoalkyle en C₁-C₄ ; alcoxy(C₁-C₄)alkyle en C₁-C₄ ; monoalkyl(C₁-C₄)aminoalkyle en C₁-C₄ ; dialkyl(C₁-C₄)aminoalkyle en C₁-C₄ ; aminocarboxyalkyle en C₁-C₄ ; acétyl alkyl(C₁-C₄)aminoalkyle en C₁-C₄ ; ou un cycle aromatique non substitué ou substitué par un ou plusieurs radicaux choisis parmi un atome d'halogène, un radical alkyle en C₁-C₄, trifluorométhyle, alcoxy en C₁-C₄, amino, et amino mono- ou disubstitué par un radical alkyle en C₁-C₄, par un radical alkyl(C₁-C₄)aminoalkyle en C₁-C₄, par un radical dialkyl(C₁-C₄)aminoalkyle en C₁-C₄, par un radical carboxyle ou par un radical sulfoxy ;
R₃ peut également représenter un hétérocyclique insaturé à 5 ou 6 chaînons ;
- R₂ et R₄, identiques ou différents, représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄, monoalkyl(C₁-C₄)aminoalkyle en C₁-C₄, dialkyl(C₁-C₄)aminoalkyle en C₁-C₄, carboxy, cyano, carboxyalkyle en C₁-C₄, nitro, ou un cycle aromatique à 5 ou 6 chaînons non substitué ou substitué par un ou plusieurs radicaux choisis parmi un atome d'halogène, un radical alkyle en C₁-C₄, trifluorométhyle, alcoxy en C₁-C₄, amino, et amino mono- ou disubstitué par un radical alkyle en C₁-C₄, par un radical alkyl(C₁-C₄)aminoalkyle en C₁-C₄, par un radical dialkyl(C₁-C₄)aminoalkyle en C₁-C₄, par un radical carboxyle ou par un radical sulfoxy ;
étant entendu qu'au moins un des radicaux R₁ et R₃ représente un atome d'hydrogène ;
- et au moins une base d'oxydation.

2. Composition selon la revendication 1, caractérisée par le fait que les cycles aromatiques de la formule (I) sont choisis parmi les cycles phényle, nitrophényle, alkylphényle, alcoxyphényle, polyalkylphényle, et polyalcoxyphényle.

3. Composition selon la revendication 1 ou 2, caractérisée par le fait que les hétérocycles insaturés à 5 ou 6 chaînons pouvant être représentés par le radical R₃ sont choisis parmi les cycles pyrrolique, pyridinique, pyrimidinique, imidazolique, pyrazolique, oxazolique, thiazolique, triazolique, pyrazolotriazolique, pyrazoloimidazolique, pyrrolotriazolique, pyrazolopyrimidinique, pyrazolopyridinique, benzoimidazolique, benzoxazolique, benzothiazolique, indolique, indolinique, indolidinique, isoindolidinique, benzotriazolinique, pyrazinique, oxazinique, triazinique, quinolinique, tetrahydroquinolinique, benzoimidazolidinique, et benzopyrimidinique.

4. Composition selon l'une quelconque des revendications précédentes, caractérisée par le fait que les dérivés d'indolizine de formule (I) sont choisis parmi:
- l'indolizine ;
- la 2-méthyl-1-phényl-indolizine ;
- la 2,8-diméthyl-indolizine ;
- l'ester méthylique de l'acide 2-méthyl-indolizine-6-carboxylique ;
- la 2,7-diméthyl-indolizine ;
- la 2-méthyl-1-(4-nitro-phényl)-indolizine ;
- la 8-méthyl-2-phényl-indolizine ;
- la 2-méthyl-indolizine ;
- la 7-méthyl-2-phényl-indolizine ;
- la 2-phényl-indolizine ;
- la 7-méthyl-2-(3-nitro-phényl)-indolizine ;
- la 7-méthyl-2-(2,5-diméthoxyphényl)-indolizine ;
- la 2-(2-pyridinyl)-indolizine ;
- la 2-(4-N,N-diméthylamino-phényl)-indolizine ;
- la 2-phényl-3-[2-(4-pyridyl)éthyl]-indolizine ;
- la 2-phényl-3-[2-(2-pyridyl)éthyl]-indolizine ;
- la 2-éthyl-7-méthoxy-indolizine ;
- la 2-éthyl-8-méthoxy-indolizine ;
- l'éthyl ester d'acide 2-butyl-7-indolizine-carboxylique ;
- la 1-éthyl-indolizine ;
- la 6,8-diméthyl-indolizine ;
- la 5-éthyl-7-méthyl-indolizine ;
- la N,N-diéthyl-2-méthyl-1-indolizine-éthanamine ;
- la N,N-diméthyl-2-méthyl-1-indolizine-éthanamine ;
- la N-éthyl-2-méthyl-1-indolizine-éthanamine ;
- la N-méthyl-2-méthyl-1-indolizine-éthanamine ;
- la -2-méthyl-1-indolizine-éthanamine ;
- la 5,7-diméthyl-indolizine ;
- la 8-cyano-2-méthyl-indolizine ;
- la 1-éthyl-2-méthyl-indolizine;
- l'acide 5-indolizineacétique ;
- la dihydro-indolizine ;
- la 1-indolizine-alanine
- la 6-éthyl-1,2-diméthyl-indolizine ;
- la 3-éthyl-indolizine ;
- la 2-éthyl-1-méthyl-indolizine ;
- la N,N-diéthyl-2-méthyl-3-indolizine-éthanamine ;
- la N-éthyl-2-méthyl-3-indolizine-éthanamine ;
- la N-éthyl-N-[2-(2-méthyl-3-indolizinyl)éthyl]-acétamide ;
- la N-[2-(2-méthyl-3-indolizinyl)éthyl]-acétamide ;
- la N,N,2-triméthyl-3-indolizine-éthanamine ;
- la N,2-diméthyl-3-indolizine-éthanamine ;
- la 2-méthyl-3-indolizine-éthanamine ;
- l'éthyl ester d'acide 7-indolizine carboxylique ;
- l'éthyl ester d'acide 6-indolizine carboxylique ;
- la N,N-diéthyl-2-méthyl-3-indolizine-propanamine ;
- la N-éthyl-N-[3-(2-méthyl-3-indolizinyl)propyl]-acétamide ;
- la N-éthyl-2-méthyl-3-indolizine-propanamine ;
- la N-[3-(2-méthyl-3-indolizinyl)propyl]-acétamide ;
- la N,N,2-triméthyl-3-indolizine-propanamine ;
- la N,2-diméthyl-3-indolizine-propanamine ;
- la 2-méthyl-3-indolizine-propanamine ;
- la 2-méthyl-8-nitro-indolizine ;
- la 2-méthyl-6-nitro-indolizine ;
- la N,N'-diméthyl-1,2-indolizine-éthanamine ;
- le méthyl ester d'acide 2-méthyl-7-indolizine-carboxylique ;
- l'éthyl ester d'acide 2-méthyl-7-indolizine-carboxylique ;
- la 2-méthyl-7-indolizine-carboxamide ;
- la N-éthyl-2-indolizine-éthanamine ;
- la N-méthyl-2-indolizine-éthanamine ;
- l'éthyl ester d'acide 2-méthyl-8-indolizine-carboxylique ;
- l'acide 2-méthyl 8-indolizine-carboxylique ;
- la 2-méthyl-6-méthoxy-indolizine ;
- la 2,8-diméthyl-indolizine ;
- la 8-méthyl-indolizine ;
- la N-[(2-méthyl-7-indolizinyl)méthyl]-acétamide ;
- la 7-(aminométhyl)-2-méthyl-indolizine ;
- la 2-méthyl-7-indolizine-carboxamide ;
- la 1-β-hydroxyéthyl-2-méthyl-indolizine ;
- la 6-hydroxyméthyl-2-méthyl-indolizine ;
- l'éthyl ester de 6-carboxy-2-méthyl-indolizine ;
- la 1-hydroxyméthyl-indolizine ;
- la 2-aminométhyl-indolizine ;
- la 2-hydroxyméthyl-indolizine ;
- la 2,3,7-triméthyl-indolizine ;
- la 2,3,6-triméthyl-indolizine ;
- la 5-méthyl-indolizine ;
- la 3,5-diméthyl-indolizine ;
- la 1-méthyl-indolizine ;
- la 2-méthyl-indolizine ;
- la 7-méthyl-indolizine ;
- la 6-méthyl-indolizine ;
- la 3-méthyl-indolizine ;
- la 6-éthyl-2,3-diméthyl-indolizine ;
- la 6-éthyl-2-méthyl-indolizine ;
- la 1,2-diméthyl-indolizine ;
- la 2,7-diméthyl-indolizine ;
- la 2,6-diméthyl-indolizine ;
- la 2,5-diméthyl-indolizine ;
- la 2,3-diméthyl-indolizine :;
- la 2-(3-furanyl)-indolizine ;
- la 2-(2-thiazolyl)-indolizine ;
- la 2-(3-thiényl)-indolizine ;
- la 2-(2-thiényl)-indolizine ;
- la 5,7-diméthyl-2-phényl-indolizine ;
- la 2-(3,4-diméthoxyphényl)-indolizine ;
- la 5-carboxy-2-phényl-indolizine ;
- l'acide 4-(7-indolizinyl)-benzène-sulfonique ;
- l'acide 4-(2-indolizinyl)-benzène-sulfonique ;
- la 3,8-diméthyl-2-phényl-indolizine ;
- la 3,7-diméthyl-2-phényl-indolizine ;
- la 3,6-diméthyl-2-phényl-indolizine ;
- la 3,5-diméthyl-2-phényl-indolizine ;
- la 7-méthyl-8-nitro-2-phényl-indolizine ;
- la 7-méthyl-6-nitro-2-phényl-indolizine ;
- la 1-β-aminoéthyl-2-phényl-indolizine ;
- la 8-méthyl-2-phényl-indolizine ;
- la 2-(3-méthoxyphényl)-indolizine ;
- la 6-éthyl-2-phényl-indolizine ;
- la 2-(2,5-diméthoxyphényl)-5-méthyl-indolizine ;
- la 2-(2,5-diméthoxyphényl)-indolizine ;
- la 6-méthyl-2-phényl-indolizine ;
- la 8-carboxy-2-phényl-indolizine
- la 2-(4-γ-hydroxypropylphényl)-indolizine ;
- la 2-(4-β-hydroxyéthylphényl)-indolizine ;
- la 8-nitro-2-phényl-indolizine ;
- la 6-nitro-2-phényl-indolizine ;
- la 6-carboxy-2-phényl-indolizine ;
- la 6-carboxy-2-phényl-indolizine ;
- la 5-méthyl-2-phényl-indolizine ;
- la 7-méthoxy-2-phényl-indolizine ;
- la 1-[2-(diméthylamino)éthyl]-2-phényl-indolizine ;
- la 1-[3-(diméthylamino)propyl]-2-phényl-indolizine ;
- la 1-[(diméthylamino)méthyl]-2-phényl-indolizine ;
- la 2-(4-méthoxyphényl)-indolizine ;
et leurs sels d'addition avec un acide.

5. Composition selon l'une quelconque des revendications précédentes, caractérisée par le fait que le ou les dérivés d'indolizine de formule (I) et/ou le ou leurs sels d'addition avec un acide représentent de 0,0005 à 12 % en poids du poids total de la composition tinctoriale.

6. Composition selon la revendication 5, caractérisée par le fait que le ou les dérivés d'indolizine de formule (I) et/ou le ou leurs sels d'addition avec un acide représentent de 0,005 à 6 % en poids du poids total de la composition tinctoriale.

7. Composition selon l'une quelconque des revendications précédentes, caractérisée par le fait que la ou les bases d'oxydation sont choisies parmi les paraphénylènediamines, les bases doubles, les para-aminophénols, les ortho aminophénols et les bases d'oxydation hétérocycliques.

8. Composition selon la revendication 7, caractérisée par le fait que les paraphénylènediamines sont choisies parmi les composés de formule (II) suivante et leurs sels d'addition avec un acide : 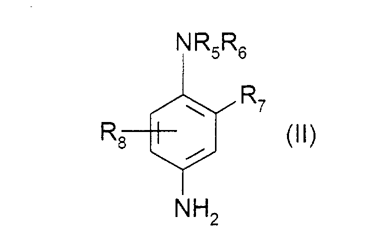 dans laquelle :
- R₅ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₆ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₇ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₈ représente un atome d'hydrogène, d'halogène ou un radical alkyle en C₁-C₄.

9. Composition selon la revendication 8, caractérisée par le fait que les paraphénylènediamines de formule (II) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl) amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

10. Composition selon la revendication 7, caractérisée par le fait que les bases doubles sont choisies parmi les composés répondant à la formule (III) suivante, et leurs sels d'addition avec un acide : 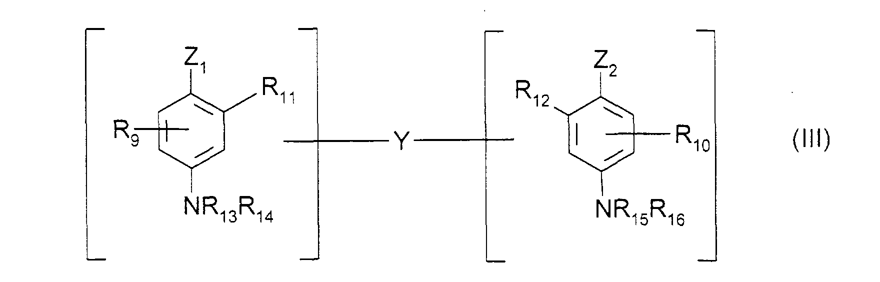 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₉ et R₁₀ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₁, R₁₂, R₁₃, R₁₄, R₁₅ et R₁₆, identiques ou différents, représentent un atome d'hydrogène, un bras de liaison Y ou un radical alkyle en C₁-C₄ ;
étant entendu que les composés de formule (III) ne comportent qu'un seul bras de liaison Y par molécule.

11. Composition selon la revendication 10, caractérisée par le fait que les bases doubles de formules (III) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

12. Composition selon la revendication 7, caractérisée par le fait que les para-aminophénols sont choisis parmi les composés répondant à la formule (IV) suivante, et leurs sels d'addition avec un acide : 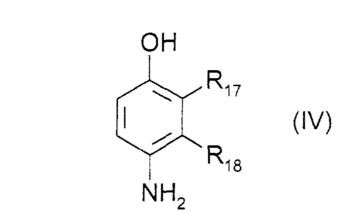 dans laquelle :
- R₁₇ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₈ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₇ ou R₁₈ représente un atome d'hydrogène.

13. Composition selon la revendication 12, caractérisée par le fait que les para-aminophénols de formule (IV) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

14. Composition selon la revendication 7, caractérisée par le fait que les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

15. Composition selon la revendication 7, caractérisée par le fait que les bases hétérocycliques sont choisies parmi les dérivés pyridiniques, les dérivés pyrimidiniques, les dérivés pyrazoliques, et leurs sels d'addition avec un acide.

16. Composition selon l'une quelconque des revendications précédentes, caractérisée par le fait que la ou les bases d'oxydation représentent de 0,0005 à 12 % en poids du poids total de la composition tinctoriale.

17. Composition selon la revendication 16, caractérisée par le fait que la ou les bases d'oxydation représentent de 0,005 à 6 % en poids du poids total de la composition tinctoriale.

18. Composition selon l'une quelconque des revendications précédentes, caractérisée par le fait qu'elle renferme un ou plusieurs coupleurs additionnels et/ou un ou plusieurs colorants directs.

19. Composition selon la revendication 18, caractérisée par le fait que le ou les coupleurs additionnels sont choisis parmi les métaphénylènediamines, les méta-aminophénols, les métadiphénols, les coupleurs hétérocycliques, et leurs sels d'addition avec un acide.

20. Composition selon l'une quelconque des revendications précédentes, caractérisée par le fait que les sels d'addition avec un acide sont choisis parmi les chlorhydrates, les bromhydrates, les sulfates et les tartrates, les lactates et les acétates.

21. Utilisation des dérivés d'indolizine de formule (I), tels que définis à l'une quelconque des revendications 1 à 4 et 20, à titre de coupleur, en association avec au moins une base d'oxydation, pour la teinture d'oxydation des fibres kératiniques et en particulier des fibres kératiniques humaines telles que les cheveux.

22. Procédé de teinture d'oxydation des fibres kératiniques et en particulier des fibres kératiniques humaines telles que les cheveux, caractérisé par le fait que l'on applique sur lesdites fibres au moins une composition tinctoriale telle que définie à l'une quelconque des revendications 1 à 20, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

23. Procédé selon la revendication 22, caractérisé par le fait que l'agent oxydant est choisi parmi le peroxyde d'hydrogène, le peroxyde d'urée, les bromates de métaux alcalins, les persels, les peracides et les enzymes.

24. Dispositif à plusieurs compartiments, ou "kit" de teinture à plusieurs compartiments, dont un premier compartiment renferme une composition tinctoriale telle que définie à l'une quelconque des revendications 1 à 20 et un second compartiment renferme une composition oxydante.

25. Dérivés d'indolizine choisis parmi :
- l'ester méthylique de l'acide 2-méthyl-indolizine-6-carboxylique ;
- la 7-méthyl-2-(2,5-diméthoxyphényl)-indolizine ;
et leurs sels d'addition avec un acide.
